# EUROPEAN PATENT APPLICATION

(11) **EP 4 219 113 A1**
(43) Date of publication of application: **02.08.2023**
(21) Application number: 23153374.6
(22) Date of filing: 26.01.2023
(51) Int. Cl.: B29B 9/16, B01D 53/30, B29B 13/06, C08F 6/00, G01N 33/00

(54) **APPARATUS AND METHOD FOR TREATING INCOHERENT MATERIAL**

(30) Priority: 26.01.2022 IT 202200001325
(71) Applicant: Piovan S.P.A., 30036 Santa Maria di Sala (VE) (IT)
(72) Inventor: BELLIO, Enrico, 31050 Ponzano V.to (IT)
(74) Representative: Luppi Intellectual Property S.r.l.

(57) **Abstract**

A treatment apparatus and method are disclosed for reducing tetrahydrofuran THF from incoherent bioplastic and/or biopolymer material, with a container for containing the material, with flow generating means for generating a flow of process gas from a gas inlet to a gas outlet, with olfactory sensor means characterized for detecting the THF content in a gas and arranged to detect the THF content in the process gas exiting the container, and with control means configured to control at least one operating parameter of the process on the basis of signals supplied by the olfactory sensor means.

## Description

### Background of the invention

The invention relates to a treatment apparatus and method for processing incoherent material, in particular bioplastic material, in the form of granules and/or pellets and/or microgranules and/or powder and/or chips and/or flakes or the like.

Specifically, but not exclusively, the invention can be advantageously applied in a plant for treating the incoherent material, such as for example a dehumidification and/or drying and/or crystallization and/or vacuum and/or pressure conveying plant of the incoherent material.

This plant can be intended, in particular, to supply a user machine, like, for example, a machine for processing and transforming plastics, in particular an extruder that supplies extruded plastics to an injection and/or blow and/or compression moulding apparatus.

It is known to divide bioplastics from renewable sources into two major categories: (i) biologically based polymers deriving from renewable and biodegradable sources and (ii) polymers deriving from renewable and non-biodegradable sources.

Materials characterized by a degrading mechanism by microorganisms that cause an irreversible chemical reaction breaking old bonds and/or forming new bonds in the macromolecular chain of the polymer fall into the first category. A thermoplastic polyester obtained by polymerizing the lactic acid, for example PLA, belongs to this category.

So-called "green plastics" fall into the second category, i.e. an ecological version of commonly used polymers like polypropylene (PP), polyethylene (PE) and polyethylene terephthalate (PET). In this case, the monomers derive from bioethanol produced by fermentation of certain vegetable species. The final polymer is, in general, not biodegradable. The production cycle is nevertheless advantageous for the environmental impact because it generates a relatively low quantity of greenhouse gas.

Tetrahydrofuran THF, or oxolane, is an organic compound with the formula (CH₂)₄O, classified as a heterocyclic compound, in particular an ether cycle; at ambient temperature it is a colourless, water-mixable, low viscosity liquid; it is a relatively nontoxic and highly flammable solvent.

Tetrahydrofuran THF is a by-product of the polymerisation reaction in the production of biopolymers and remains present in the polymer granule and, moreover, can be used by the chemical and pharmaceutical industry as a solvent and/or as an intermediate product, for example to produce elastic fibres.

It is known that furans are formed from various substances that are naturally present in foodstuffs like vitamin C, carbohydrates, amino acids, unsaturated fatty acids and carotenoids. The conditions of cooking of transforming food contribute to determining how much furan is formed, or lost (mainly through evaporation) or how much of it remains at the moment of consuming the foods. It is nevertheless desirable to reduce the levels of furans present in the foodstuffs to avoid possible harm to health.

For this reason, it can be advantageous, in the plastics transforming industry, in particular in the sector of food packaging, to limit, eliminate or correct the furan in the end product. In particular, it is desirable to reduce the presence of tetrahydrofuran THF in the end products used in the packaging sector and obtained from biopolymers, to avoid the migration of tetrahydrofuran THF to the packaged foodstuffs.

It is known that biopolymers can have an amorphous structure, so the transformation process in the end product may require a further crystallisation treatment, unlike crystalline materials where, in general, only drying and/or dehumidification are required.

In a drying and/or dehumidification and/or crystallisation treatment, a drawback may be detected that arises from the tetrahydrofuran THF tendency, which has an ignition point around 60 °C, to form highly explosive mixtures.

Another drawback of the prior art lies in the fact that the production systems for producing biopolymer granule do not permit an effective reduction of the tetrahydrofuran THF content from the end product.

Patent publication EP 3 798 560 A1 shows a method and an apparatus according to the preamble of claims 1 and 8.

### Summary of the invention

One object of the invention is to propose an apparatus and/or a method that is able to overcome one or more of the aforesaid limits and drawbacks of the prior art.

One object is to provide an apparatus and/or a method for processing incoherent plastics that are alternatives to the prior art.

One object is to enable biopolymers to be produced through a process with great sustainability, oriented in particular to reducing or eliminating the tetrahydrofuran THF content from the end product.

One advantage is to provide an apparatus and/or a method that is able to reduce the tetrahydrofuran content in incoherent bioplastic material.

One advantage is to make an apparatus and/or a method available that enables incoherent plastics to be processed with precision and repeatability in order to reduce and/or eliminate the tetrahydrofuran from the material.

One advantage is to make an apparatus and/or a method for processing incoherent bioplastic material with high efficiency to reduce the furan, tetrahydrofuran or other content deriving from the furan, from the material.

One advantage is reducing or removing the tetrahydrofuran, or other furans, from bioplastic material (in particular from copolymers) in incoherent format, i.e. in granules and/or microgranules and/or powder and/or chips and/or flakes or the like.

One advantage is eliminating or reducing the substances that cause the formation of tetrahydrofuran from the incoherent plastics.

One advantage is permitting a measurement of the amount of residual tetrahydrofuran in the processed material and/or the amount of tetrahydrofuran extracted from the processed material, where the amount of residual or extracted tetrahydrofuran can be expressed, in particular, in ppm and/or mg/kg, in relation to the amount of processed material.

One advantage is controlling securely and reliably a process of reducing the tetrahydrofuran in polymer granules from biomaterials.

One advantage is permitting the incoherent bioplastic material to be processed, in particular to reduce and/or eliminate tetrahydrofuran from the material, with relatively high energy efficiency.

Such objects and advantages, and still others, are achieved by a method and/or an apparatus according to one or more of the claims set out below.

In one embodiment, a treatment apparatus, in particular for reducing or extracting or eliminating tetrahydrofuran, comprises a container for containing incoherent plastics, flow generating means for generating a flow of process gas from a gas inlet to a gas outlet of the container, olfactory sensor means characterized for detecting the tetrahydrofuran THF content in a gas and arranged to detect the tetrahydrofuran THF content in the process gas exiting the container, and control means configured to control at least one operating parameter of the process on the basis of signals supplied by the olfactory sensor means.

The aforesaid operating parameter of the process may comprise, in particular, one or more of the following parameters: an indicative parameter of the humidity of the process gas entering the container, an indicative parameter of the flowrate of the process gas entering and/or exiting the container, an indicative parameter of the humidity content of the incoherent plastics (inside, at the inlet or at the outlet of the container), an indicative parameter of an operating intensity of means for mixing the material inside the container, an indicative parameter of the temperature of the process gas entering the container, an indicative parameter of an operating intensity of means for recirculating the material from an outlet to an inlet of the container.

### Brief description of the drawings

The invention can be better understood and implemented with reference to the attached drawings that illustrate some embodiments thereof by way of non-limiting example, in which:
Figure 1 shows a vertical raised drawing of a first embodiment of a treatment apparatus for processing incoherent plastics, made in accordance with the present invention, in particular for extracting and/or reducing and/or removing the tetrahydrofuran content from the material;
Figure 2 shows a vertical raised drawing of a second embodiment of a treatment apparatus for processing incoherent plastics, made in accordance with the present invention, in particular for extracting and/or reducing and/or removing the tetrahydrofuran content from the material;
Figure 3 shows an embodiment of a tetrahydrofuran THF extraction curve from incoherent plastics in function of time;
Figure 4 shows a block diagram of an embodiment of an algorithm that is usable in a treatment method made in accordance with the present invention, in particular that is implementable on the control means of any one of the treatment apparatuses of Figures 1 and 2.

### Detailed description

With reference to the attached figures, for the sake of the simplicity of exposition, identical elements of different embodiments have been indicated by the same numbering. With reference to the aforesaid figures, with 1 a treatment apparatus has been indicated overall for processing incoherent plastics, in particular for processing incoherent bioplastic material, i.e. bioplastic material in the form of granules and/or pellets and/or microgranules and/or powder and/or chips and/or flakes or the like. The treatment apparatus 1 is used, in particular, for reducing and/or extracting and/or eliminating furan and/or tetrahydrofuran THF and/or one or more furan derivates from incoherent bioplastic and/or biopolymer material.

The treatment apparatus 1 may comprise, in particular, at least one container 2 (for example, a hopper) that is suitable for containing the incoherent material. The container 2 may comprise, in particular, at least one gas inlet 3 for the entry of a process gas and at least one gas outlet 4 for the exit of the process gas. The container 2 may comprise, in particular, at least one inlet IN for the entry of the material to be processed and at least one outlet OUT for the exit of the processed material.

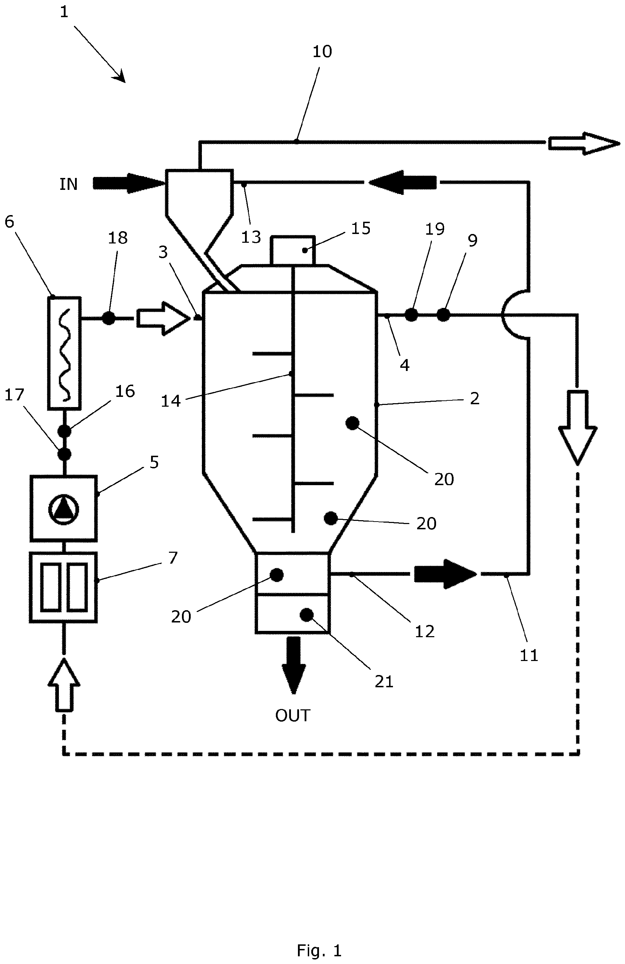

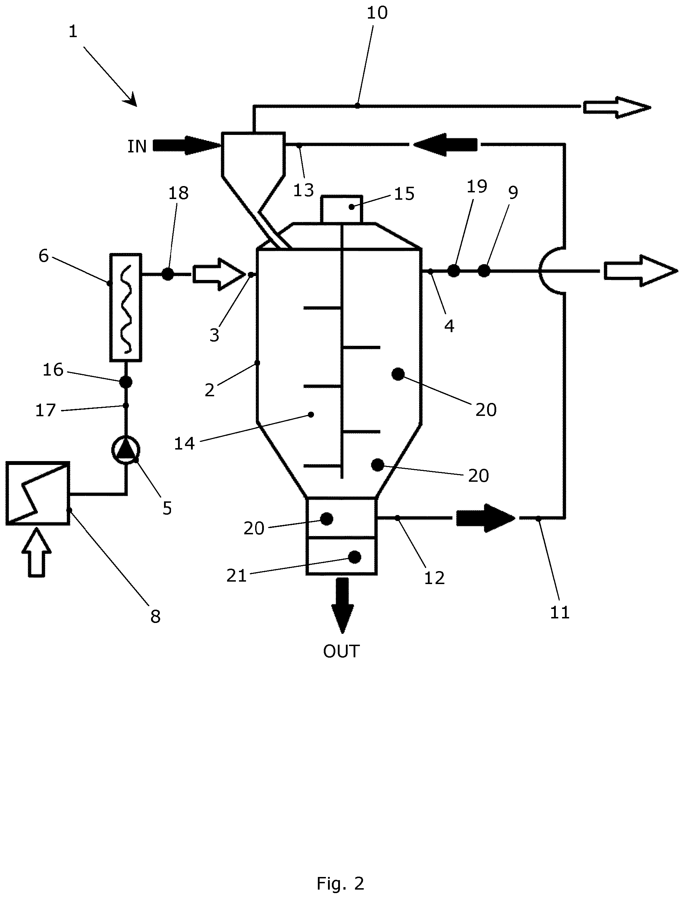

The treatment apparatus 1 may comprise, in particular, flow generating means for generating a flow of process gas from the gas inlet 3 to the gas outlet 4. The flow generating means may comprise, in particular, at least one actuator 5 (for example a pump or a fan) configured to move a flow of gas in a conduit. The flow generating means may be, in particular, configured to generate a flow in a closed circuit (in which the process gas exiting the container is treated and returned to the container), as in the embodiment in Figure 1. The flow generating means may be, in particular, configured to generate a flow in an open circuit (in which the process gas entering the container is removed from the environment and the process gas exiting the container is released into the environment), as in the embodiment in Figure 2 (suitable, in particular, for treating amorphous plastics and/or for treating crystalline plastics). It is nevertheless possible to configure the apparatus in Figure 1, with appropriate modifications, so as to generate a flow in an open circuit, and configure the apparatus in Figure 2 so as to generate a flow in a closed circuit.

The flow generating means may comprise, in particular, at least one heater 6 for heating the process gas entering the container. The flow generating means may comprise, in particular, at least one dehumidifier for dehumidifying the process gas entering the container. The dehumidifier may comprise, in particular, a dehumidifier of the type with drying materials, in particular of the type with molecular sieves, like, for example, a dual tower dehumidifier for dehumidifying molecular sieves 7 (see embodiment of Figure 1). It is possible, in particular, to use a dehumidifier with specific molecular sieves for absorbing and eliminating the tetrahydrofuran THF. The dehumidifier may comprise, in particular, a dry air generator 8 (for example a dry air generator comprising at least one gas compressor and/or a dry air generator of the type with absorption technology and/or a dry air generator of the mechanical-refrigerating type).

The treatment apparatus 1 may comprise, in particular, olfactory sensor means 9 (for example an electronic nose) characterized in that it detects at least one indicative value of the tetrahydrofuran THF content (furan or a furan derivate) in a gas. In particular, the olfactory sensor means 9 may be arranged to detect an indicative value of the THF content in the process gas exiting the container 2. The olfactory sensor means may be accordingly designed and configured and specially adapted to detect a specific substance, in particular tetrahydrofuran THF. The characterization of olfactory sensor means specifically for detecting THF in a gas is, *per se,* known and is not disclosed in greater detail.

The treatment apparatus 1 may comprise, in particular, suction means 10 configured to suck gas, in particular by means for generating a vacuum, from a top of the container 2.

The treatment apparatus 1 may comprise, in particular, control means (electronic and programmable) configured to control the treatment apparatus 1 on the basis of signals supplied by the olfactory sensor means 9. The control means may be, in particular, configured to control at least one indicative parameter of the humidity content of the process gas that enters the container 2 on the basis of signals supplied by the olfactory sensor means 9. The control means may be, in particular, configured to decrease the humidity content of the process gas that enters the container 2 if the olfactory sensor means 9 detects an increase in the tetrahydrofuran THF content in the process gas exiting the container 2. The aforesaid indicative parameter of the humidity content of the process gas may comprise, in particular, the dewpoint and/or the relative humidity and/or the specific humidity and/or the absolute humidity of the process gas.

The control means may be, in particular, configured to control at least one temperature of the process gas on the basis of signals supplied by the olfactory sensor means 9. In particular, the control means may be configured to control at least one temperature of the process gas entering the container 2 on the basis of signals supplied by the olfactory sensor means 9. The control means may be, in particular, configured to increase the temperature of the process gas that enters the container 2 if the olfactory sensor means 9 detects an increase in the tetrahydrofuran THF content in the process gas exiting the container 2. The control means may be, in particular, configured to control at least one flowrate of the process gas entering the container 2 on the basis of signals supplied by the olfactory sensor means 9. The control means may be, in particular, configured to control at least one flowrate of the process gas exiting the container 2 on the basis of signals supplied by the olfactory sensor means 9. The control means may be, in particular, configured to increase the flowrate of the process gas that enters the container 2 if the olfactory sensor means 9 detects an increase in the tetrahydrofuran THF content in the process gas exiting the container 2. The control means may be, in particular, configured to receive data relating to the incoherent plastics to be processed and to control the flow generating (in particular, the heater 6) on the basis of the aforesaid data received on the material and on the basis of signals supplied by the olfactory sensor means 9.

The control means may be, in particular, configured to control at least one indicative parameter of the humidity content of the incoherent plastics on the basis of signals supplied by the olfactory sensor means 9. The control means may be, in particular, configured to decrease the humidity content of the plastics exiting the container 2 if the olfactory sensor means 9 detects an increase in the tetrahydrofuran THF content in the process gas exiting the container 2.

The treatment apparatus 1 may comprise, in particular, recirculating means 11 configured to recirculate at least one part of the incoherent plastics from an outlet 12 to an inlet 13 of the container. The recirculating means 11 may comprise, in particular, at least one conveyor of incoherent plastics. The control means may be, in particular, configured to control the recirculating means 11 on the basis of signals supplied by the olfactory sensor means 9. The control means may be, in particular, configured to increase an operating speed of the recirculating means 11 if the olfactory sensor means 9 detects an increase in the tetrahydrofuran THF content in the process gas exiting the container 2.

The treatment apparatus 1 may comprise, in particular, mixing means 14 configured to mix the incoherent plastics in the container 2. The mixing means 14 may comprise, in particular, a rotating shaft (with a vertical axis) provided with blades and driven by a motor 15. The control means may be, in particular, configured to control the mixing means 14 on the basis of signals supplied by the olfactory sensor means 9. The control means may be, in particular, configured to increase an operating speed of the mixing means 14 if the olfactory sensor means 9 detects an increase in the tetrahydrofuran THF content in the process gas exiting the container 2.

The treatment apparatus 1 may comprise, in particular, at least one humidity sensor 16 arranged to detect a value (for example the dewpoint) indicative of the humidity in the process gas. The treatment apparatus 1 may comprise, in particular, at least one flowrate sensor 17 arranged to detect the flowrate of the process gas. The treatment apparatus 1 may comprise, in particular, at least one temperature sensor 18 arranged to detect the temperature of the process gas at the inlet 3 of the container 2. The treatment apparatus 1 may comprise, in particular, at least one temperature sensor 19 arranged to detect the temperature of the process gas at the outlet 4 of the container 2. The treatment apparatus 1 may comprise, in particular, one or more temperature sensors 20 arranged to detect the temperature of the incoherent plastics in one or more zones of the container 2, for example near the outlet OUT and/or in one or more intermediate zones of the container 2. The treatment apparatus 1 may comprise, in particular, at least one humidity sensor 21 arranged to detect an indicative parameter of the humidity content of the incoherent material in the container 2, for example near the outlet OUT of the container 2.

The treatment apparatus 1 in particular enables a treatment method to be actuated that may comprise the step of generating a flow of process gas through the incoherent plastics. The treatment method may comprise, in particular, the step of detecting by the olfactory sensor means 9 the tetrahydrofuran THF content in the process gas that has traversed the incoherent plastics. The treatment method may comprise, in particular, the step of controlling at least one operating parameter of the method on the basis of the aforesaid detected content. The aforesaid operating parameter may comprise, in particular, at least one parameter selected from the group comprising: an indicative parameter of the humidity content of the process gas, an indicative parameter of a temperature of the process gas, an indicative parameter of a flowrate of the process gas, an indicative parameter of the humidity content of the incoherent plastics, an indicative parameter of a mixing speed of the incoherent plastics, an indicative parameter of a recirculating speed of the incoherent plastics.

It is possible to provide at least two different methods of extraction of the THF from the incoherent plastics. A first method can be particularly advantageous for processing plastics (for example a biocopolymer) with amorphous structure. A second method can be particularly advantageous for processing plastics (for example a biocopolymer) with a crystalline structure. Both methods can be performed with a continuous process or with a batch process. With both methods, it is possible to mix the material during treatment. With both methods, it is possible to use a process gas (for example, air), which can be heated to a desired temperature according to the features of the material.

In one embodiment, a treatment method of the incoherent plastics, in particular for reducing and/or eliminating the tetrahydrofuran THF from the material, may comprise the step of generating a flow of a process gas through the incoherent plastics to extract the tetrahydrofuran THF. The treatment method may comprise, in particular, the step of detecting (by the olfactory sensor means 9) possible odour emissions of tetrahydrofuran THF in the process gas that has traversed the incoherent plastics. The treatment method may comprise, in particular, the step of controlling at least one process operating parameter on the basis of the odour emissions detected. This process operating parameter may comprise, in particular, an indicative parameter of the humidity content of the process gas and/or an indicative parameter of the temperature of the process gas and/or an indicative parameter of the flowrate of the process gas and/or other parameters as disclosed above. This process operating parameter could, however, comprise another process operating parameter, in particular an indicative parameter of a chemical-physical feature of the process gas and/or an indicative parameter of a feature of the incoherent material and/or an indicative parameter of an operating feature of an actuator.

The treatment method can, in particular, control one or more different process parameters, for example depending on the features of extraction and/or reduction of the tetrahydrofuran THF from the plastics. In particular, in the case of amorphous material, a thermal flow can be provided so as to generate a heat flow so as to prevent softening of the material and resulting sticking, in particular taking account of the specific glass transition temperature T_{g} that will have to pass through the processed material. It is possible to control appropriately the speed of the rotation axis of the mixing means 14 of the material and/or the flowrate of the process gas, so as to avoid softening (even local) of the material being treated.

The olfactory sensor means 9 that detects the tetrahydrofuran THF content in the process gas, and which can be arranged, in particular, at the outlet of the container 2, enables possible modifications to be detected in the activity of extracting tetrahydrofuran THF and enables the (electronic) control means to establish an appropriate variation in the setpoint values of the control of one or more actuators.

The method can comprise, in particular, the step of controlling at least one indicative parameter of the humidity of the processed material (in particular the degree of final humidity of the granule) and of comparing this indicative parameter of the humidity of the material with the tetrahydrofuran THF value present in the process gas and detected by the olfactory sensor means 9 arranged at the outlet of the container 2. The method may comprise, in particular, the step of recirculating the material by removing the material (bioplastic granule) from a (lower) outlet 12 of the container 2 and returning the material to an (upper) inlet 13 of the container, and the step of controlling the recirculating means 11 to recirculate the material on the basis of the tetrahydrofuran THF value in the process gas measured by the olfactory sensor means 9, so as to prevent, on one side inappropriately processed material being processed (for example material at an inappropriate temperature), and on the other side, material being recirculated that has remained exposed to the heat process too long with too dry a flow of process gas (for example a gas with too negative a dewpoint).

As said, the treatment method can be diversified according to the structure of the polymer treated, in particular for an amorphous structure of the incoherent material and for a crystalline structure of the incoherent material. A treatment method particularly suitable for processing bioplastic material with amorphous structure may comprise, in particular, a material crystallisation process step. The crystallisation step may comprise, in particular, the step of providing a container (crystalliser) provided with a mixing system with a mixing axis for maintaining the incoherent material in movement and avoiding the formation of agglomerates due to the transition of the material being treated through the glass transition temperature T_{g}. A treatment method that is suitable for processing bioplastic material with both an amorphous structure and with a crystalline structure may comprise, in particular, a material recirculating process step. The recirculating step may involve, in particular, removing a set quantity of material from a discharge of the container 2 (hopper), in particular at a set frequency, to then return the removed quantity of material to an inlet of the container. The recirculating step can be used, in particular, to standardize the thermal profile of the material (plastic granule obtained from a biopolymer) inside the container 2.

The method can, in particular, activate recirculating of the material after a certain time from the start of the plastics treatment step, in particular after a certain time from the start of the introduction of the process gas into the material contained in the container 2, to ensure a suitable time for heating the material before extraction thereof for recirculating and permitting subsequent extraction of the THF from the material in the upper part of the container.

The method (in particular with crystallisation and/or recirculation) may comprise, in particular, using a process gas that comprises only heated or dried air, or dehumidified air. In both cases it is possible to operate in open circuit so as to prevent a concentration of the extracted substance (furan and/or THF and/or furan derivates) in the inner parts of the system.

The treatment apparatus may comprise, in particular, one or more intercepting means for intercepting the incoherent material and/or containing the incoherent material. It is possible for each of these intercepting members to be so shaped and sized as to prevent material collecting zones that could cause the formation of explosive mixtures.

A treatment method that is suitable for processing bioplastic material with a crystalline structure, where the crystallisation process step is not necessary, may comprise, in particular, the operation of maintaining the material in movement, for example with a recirculating step and/or a mixing step in order to standardize the temperature of the material to homogenize the extraction of the tetrahydrofuran.

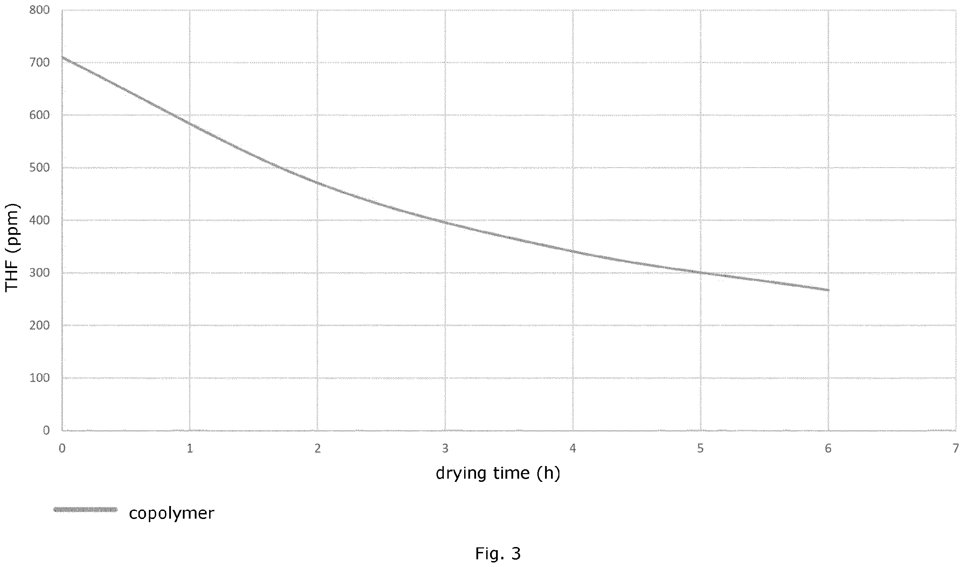

Figure 3 shows an embodiment merely by way of non-limiting example of an extraction curve of the tetrahydrofuran THF from a bipolymer with polypropylene structure. An extraction curve can in particular be understood to be a trend over time of the tetrahydrofuran THF content value detected in the process gas that has processed the material. The test was conducted by treatment with a crystallisation system provided with an air drying (heating) unit. The system provided a flow of heated air in open circuit and with a material recirculating device. The treatment enabled the tetrahydrofuran THF to be reduced by 60% after 6 hours of treatment of the plastic granule: from an initial measured value of 725 ppm, 267 ppm were reached after 6 hours of treatment.

It was also observed experimentally that the action of dehumidifying the process gas facilitates and significantly improves the activity of extracting the tetrahydrofuran THF. Tests conducted show that some process operating parameters can affect the tetrahydrofuran THF extraction speed and/or efficiency, like, for example, the duration of the treatment, the temperature of the process gas and the flowrate of the process gas. The method may comprise, in particular, detecting the quantity of tetrahydrofuran THF extracted from the material by monitoring the flow of process gas, measuring the tetrahydrofuran THF content in the process gas in various moments of the process: the greater the difference of the THF content between two successive instants the greater the efficiency of the system will be. It has been further found that the temperature of the process gas is a critical parameter that has to be checked carefully and cannot exceed a certain threshold of the flammability limits of the THF and/or the thermal degradation limits of the material.

The duration of the treatment influences extraction of the THF from the material, although it has been observed that after a certain extraction time the extraction curve (i.e. the trend over time of the tetrahydrofuran THF content value in the process gas that has processed the incoherent material) tends to flatten, in particular it flattens along an asymptotic tangent. For this reason, it is possible to use the treatment for a limited time.

The flowrate of the process gas may be, in particular, a process parameter that may be modified, in particular, at the same time as the change of the temperature of the process gas, during advancement of the treatment time. In particular, it is possible to increase the flowrate of the process gas together with the increase of the temperature of the process gas.

The method in question comprises a control algorithm that may comprise, in particular, a step of checking the extraction curve gradient of the THF (this checking step may be repeated, in particular, periodically at set intervals of time), where the "gradient" may be evaluated as a quotient between the difference in the THF value measured in two subsequent instants and the period of time elapsing between the two measuring instants. The control algorithm may comprise, in particular, an increase step of both the temperature and the flowrate of the process gas if the extraction curve gradient is relatively very high (for example, greater than a set value Δsup), an increase step of the flowrate of the process gas without increasing the temperature of the process gas if the extraction curve gradient is relatively high (for example, greater than a set value Δinf and less than the aforesaid value Δsup with Δinf < Δsup), a step of maintaining the temperature and of the flowrate of the process gas at the preceding value if the extraction curve gradient is relatively low (for example, below the aforesaid value Δinf). It has been found that it is possible to increase the flowrate of the process gas without increasing the temperature of the process gas because temperature is a variable that should be modified only after a specific check of extraction intensity.

The control algorithm may comprise, in particular, the step of not altering either the flowrate or the temperature of the process gas if the THF extraction curve tends to maintain a constant gradient. The method in question may comprise, in particular, a control algorithm that comprises the step of controlling the dewpoint of the process gas so as to promote extraction and/or reduction of the THF and reaching a given degree of dehumidification of the plastics, in particular when a subsequent transformation process is required. The control algorithm may comprise, in particular, the step of increasing the dewpoint of the process gas that enters the container 2 (so as to increase the humidity content in the process gas) if the extraction curve gradient is greater than a set value, for example to the aforesaid value Δinf or to the aforesaid value Δsup. The control algorithm may comprise, in particular, the step of decreasing the dewpoint of the process gas that enters the container 2 (so as to decrease the humidity content in the process gas) if the extraction curve gradient is lower than a set value, for example the aforesaid value Δinf or the aforesaid value Δsup.

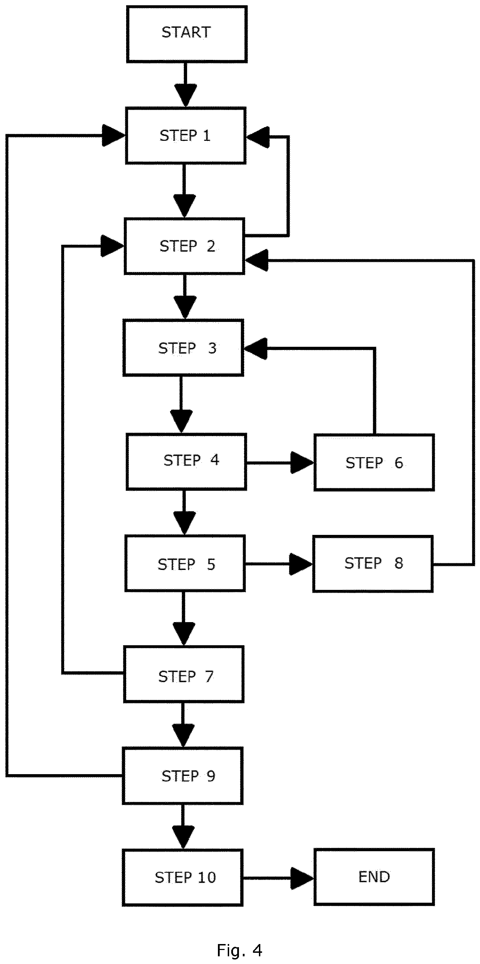

The method may comprise, in particular, steps of governing one or more process parameters (relating to the tetrahydrofuran THF elimination and/or reduction process) according to the following embodiment, indicating a possible control algorithm to implement the method, with reference to the block diagram of Figure 4.

### Treatment Embodiment

This purely illustrative and non-limiting embodiment of the invention can be particularly suitable for processing incoherent bioplastic material in an amorphous state. Initially, at least the following setpoint values are set to control the apparatus: a dewpoint value DP of the process gas entering the container, a temperature value TG of the process gas entering the container, a flowrate value Q of the process gas entering the container, a THF value indicating the desired tetrahydrofuran content in the process gas detectable by the olfactory sensor means at the outlet of the container, two values Linf and Lsup that constitute the lower and upper limit of an acceptable interval of the desirable value of the tetrahydrofuran content around the THF value (Linf < THF < Lsup), a value tlim of a set time deemed to be appropriate for performing tetrahydrofuran reduction treatment, a minimum threshold value DPlim of the dewpoint of the process gas, a minimum temperature threshold value Tlim for recirculating the processed material (where Tlim depends at least on the type of material, in particular Tlim can be greater than the glass transition T_{g} temperature of the material). This embodiment is illustrated schematically by the block diagram of Figure 4. When the incoherent plastics have been introduced into the container (for example according to a method of batch type), step 1 is proceeded to.

Step 1: recirculating of the plastics introduced into the container is maintained inactive or deactivated; the incoherent plastics introduced into the container are processed by the process gas characterized by the aforesaid setpoint values DP, TG and Q; after a set interval of time Δt, step 2 is proceeded to.

Step 2: the temperature TM of the material is measured; if TM ≤ Tlim, step 1 is returned to; if TM > Tlim, step 3 is proceeded to.

Step 3: recirculation of the material is activated or continued and the indicative current value of the tetrahydrofuran content in process gas exiting the container is measured (by the olfactory sensor means 9) and this measured current tetrahydrofuran value is stored; after a set time Δt, step 4 is proceeded to.

Step 4: the current tetrahydrofuran value in the process gas exiting the container is again measured and stored; if the current tetrahydrofuran value has decreased from the last previously measured value, then step 5 is proceeded to; if the current tetrahydrofuran value is the same as or greater than the last previously measured value, then the step 6 is proceeded to.

Step 5: if the extraction speed is very low, in particular if the extraction curve gradient of the tetrahydrofuran is very low (for example, ΔTHF < Δinf, where ΔTHF is the difference between the last two measured values of tetrahydrofuran possibly parameterised on the time elapsing between the two measurements, and Δinf is a reference value), both the temperature and the flowrate of the process gas are increased (increasing the setpoint value of the temperature T and of the flowrate Q of the process gas of set values ΔT and ΔQ, i.e. T = T + ΔT and Q = Q + ΔQ) and step 7 is proceeded to; if the extraction speed is not very low but is not sufficiently high, in particular if the extraction curve gradient of the tetrahydrofuran is less low than the preceding case (for example, Δinf < ΔTHF < Δsup, where Δsup is a reference value), then only the flowrate of the process gas (Q = Q + ΔQ) is increased and step 7 is proceeded to; if the extraction speed is relatively high, in particular if the extraction curve gradient of the tetrahydrofuran is higher than the preceding case (for example, ΔTHF > Δsup), then step 8 is proceeded to.

Step 6: the setpoint value of the flowrate Q of the process gas is increased by a set value ΔQ, i.e. Q = Q + ΔQ; after which, only if Q + ΔQ > Qlim, then Q = Qlim is set; after which step 3 is returned to.

Step 7: if the measured current tetrahydrofuran value is less than or the same as Linf (i.e. the desired tetrahydrofuran reduction level is reached ), then step 9 is proceeded to; if the measured current tetrahydrofuran value is greater than Linf (i.e. the desired tetrahydrofuran reduction level is not reached ), then the setpoint value of the dewpoint DP is decreased by a set value ΔDP, i.e. DP = DP - ΔDP, in this manner, the dehumidifying power of the process gas is increased (in practice, the dewpoint becomes more negative, for example, it increases from -30°C to -35°C), step 2 is returned to and the extraction process is continued with.

Step 8: the setpoint value of the dewpoint DP is decreased by a set value ΔDP, i.e. DP = DP - ΔDP (in practice, the dewpoint becomes more negative, for example, it moves from -30°C to -35°C), increasing in this manner the dehumidifying power of the process gas; only if the new value decreased by a setpoint of the dewpoint DP is less than DPlim, i.e. DP < DPlim, then DP = DPlim; after which, step 2 is returned to; this step 8 can be omitted (so that the algorithm ensures directly the return to step 2) if the material to be processed does not have to be dehumidified, in particular if the processed material exiting the apparatus in question is not directed in a continuous line to subsequent processes that require a desired level of humidity of the material.

Step 9: if t ≥ tlim, i.e. if the time t elapsing from the start of the tetrahydrofuran reduction treatment is greater than or the same as the maximum limit time tlim, then step 10 is proceeded to; if t < tlim, then step 1 is returned to.

Step 10: the tetrahydrofuran reduction cycle is finished and recirculation is then interrupted of the material that can be discharged from the container.

In the embodiments disclosed above, at least one indicative value of the furan and/or tetrahydrofuran content and/or furan derivates in the gas that has processed the material is measured. Alternatively or additionally to the step of measuring a value in the gas that has processed the material, it is possible to measure at least one indicative value of the furan and/or tetrahydrofuran content and/or furan derivates directly in the processed material from the process gas, for example from a sample of material exiting the container.

## Claims

1. Processing method for processing incoherent plastic material, said processing method comprising the step of:
- generating a gas flow to process said material;
**characterized by** comprising the steps of:
- detecting at least one value that is indicative of the furan and/or tetrahydrofuran and/or furan derivatives content in the gas that has processed said material;
- controlling at least one process parameter on the basis of said detection.

2. Method according to claim 1, wherein said at least one value that is indicative of the furan and/or tetrahydrofuran and/or furan derivatives content is detected by olfactory sensor means.

3. Method according to claim 1 or 2, wherein said at least one process parameter comprises at least one parameter selected from the group which includes: a parameter indicative of the moisture content of the process gas, a temperature of the process gas, a flow rate of the process gas, a parameter indicative of the moisture content of the incoherent plastic material, a parameter indicative of a mixing speed of the incoherent plastic material, a parameter indicative of a recirculation speed of the incoherent plastic material.

4. Method according to any one of the preceding claims, comprising the step of decreasing the humidity of the process gas as a function of said step of detecting at least one value that is indicative of the content of furan and/or tetrahydrofuran and/or furan derivatives in the gas which has processed said material.

5. Method according to any one of the preceding claims, comprising a step of determining a slope of an extraction curve of tetrahydrofuran THF and/or furan and/or furan derivatives and of controlling at least one process parameter based on said slope determination.

6. Method according to claim 5, comprising a step of increasing both a process gas temperature and a process gas flow rate if said slope of the extraction curve is lower than a predetermined value Δinf, and a step of increasing a process gas flow rate without increasing a process gas temperature if said slope of the extraction curve is higher than said predetermined value Δinf and lower than a predetermined value Δsup.

7. Method according to claim 6, comprising a step of maintaining unchanged the temperature and the flow rate of the process gas if said slope of the extraction curve is higher than said predetermined value Δsup.

8. Processing apparatus (1) for processing incoherent plastic material, said processing apparatus comprising:
- a container (2) to contain the material, said container comprising at least one gas inlet (3) and at least one gas outlet (4);
- flow generation means for generating a process gas flow from said gas inlet (3) to said gas outlet (4);
**characterized by** comprising:
- olfactory sensor means (9) characterized for detecting at least one indicative value of the content of furan and/or tetrahydrofuran and/or a furan derivative in a gas, said olfactory sensor means (9) being arranged to detect said value indicative of the content of furan and/or tetrahydrofuran and/or a furan derivative in the process gas leaving said container (2);
- control means configured to control said processing apparatus (1) on the basis of signals provided by said olfactory sensor means (9).

9. Apparatus according to claim 8, wherein said control means is configured to control at least one parameter indicative of the moisture content of the process gas entering said container (2) based on signals provided by said olfactory sensor means (9); said at least one indicative parameter of the moisture content of the process gas including, in particular, the dewpoint and/or relative humidity and/or specific humidity and/or absolute humidity.

10. Apparatus according to claim 8, wherein said control means is configured to decrease the moisture content of the process gas entering said container (2) if said olfactory sensor means (9) detect an increase in said value in the process gas leaving said container (2).

11. Apparatus according to any one of claims 8 to 10, wherein said control means is configured to control at least one process gas temperature, in particular at least one process gas temperature entering said container, based on signals provided by said olfactory sensory means (9).

12. Apparatus according to any one of claims 8 to 11, wherein said control means is configured to control at least one flow rate of the process gas entering said container (2) and/or exiting said container (2) based on signals provided by said olfactory sensor means (9).

13. Apparatus according to any one of claims 8 to 12, wherein said flow generation means comprises at least one heater (6) of the process gas entering said container (2); said control means being configured, in particular, to receive data relating to the incoherent plastic material to be processed and to control said at least one heater (6) on the basis of said received data and/or on the basis of signals provided by said olfactory sensor means ( 9).

14. Apparatus according to any one of claims 8 to 13, wherein said control means is configured to control at least one parameter indicative of the moisture content of the incoherent plastic material based on signals provided by said olfactory sensor means (9).

15. Apparatus according to any one of claims 8 to 14, comprising recirculation means (11) configured to recirculate at least a part of the incoherent plastic material from an outlet (12) to an inlet (13) of said container and/or stirring means (14) configured to stir the incoherent plastic material in said container (2); said control means being configured to control said recirculation means (11) and/or said stirring means (14) on the basis of signals provided by said olfactory sensor means (9).
